# EUROPEAN PATENT APPLICATION

(11) **EP 4 650 747 A1**
(43) Date of publication of application: **19.11.2025**
(21) Application number: 24891602.5
(22) Date of filing: 19.08.2024
(51) Int. Cl.: G01N 1/22, G01N 1/24, H01M 10/42, G01N 33/00, G01N 30/72, G01N 30/74

(54) **GAS COLLECTION DEVICE AND GAS ANALYSIS DEVICE USING SAME**

(30) Priority: 16.11.2023 KR 20230158770; 16.11.2023 KR 20230158776
(71) Applicant: LG Energy Solution, Ltd., Seoul 07335 (KR)
(72) Inventor: HWANG, Dongguk, Daejeon 34122 (KR); LEE, Byoungsoo, Daejeon 34122 (KR); LIM, Yeji, Daejeon 34122 (KR); SHIN, Won Kyung, Daejeon 34122 (KR); CHOI, Nak Hee, Daejeon 34122 (KR)
(74) Representative: Goddar, Heinz J.
(86) International application number: PCT/KR2024/012291
(87) International publication number: WO 2025/105653

(57) **Abstract**

The present invention relates to a gas collection device. Disclosed are a gas collection device for collecting, in real time, gas generated in a cylindrical secondary battery, and a gas analysis device using same.

## Description

The present application claims priority from Korean Patent Applications No. 10-2023-0158770 and No. 10-2023-0158776, filed on November 16, 2023, the entire disclosures of which are incorporated herein by reference in their entirety.

### Technical Field

The present disclosure relates to a gas collecting apparatus, and more particularly, to a gas collecting apparatus capable of collecting gas generated inside a secondary battery in real time and a gas analysis apparatus using same.

### Background

Secondary batteries are batteries that may be used repeatedly through a discharging process that converts chemical energy into electrical energy and a charging process that converts electrical energy into chemical energy, and commonly known types include nickelcadmium (Ni-Cd) batteries, nickel-metal hydride (Ni-MH) batteries, lithium-metal batteries, lithium-ion (Li-ion) batteries, and lithium-ion polymer batteries. Among these secondary batteries, lithium secondary batteries have been commercialized and are widely used due to their high energy density and voltage, long cycle life, and low self-discharge rate.

Various types of gases may be generated inside a lithium secondary battery depending on the charge and discharge reaction, such as hydrogen, oxygen, nitrogen, carbon monoxide, carbon dioxide, hydrocarbons such as CₙH₂ₙ₋₂ (n=2-5), CₙH₂ₙ (n=2-5), and CₙH₂ₙ₊₂ (n=1-5), and other organic gas species.

In addition, a lithium secondary battery generates a large amount of gas in the process of electrolyte decomposition and degradation due to repeated charging and discharging, and this aspect varies depending on the design and usage of the battery. Therefore, it is essential to analyze the gas generated inside the battery to infer the degradation mechanism of the battery during the battery development process.

Therefore, it is very important to collect and accurately analyze the gas generated inside the secondary battery. Information on the composition and content of various gases generated during charging and discharging of lithium secondary batteries is useful for developing battery materials, optimizing the battery manufacturing process, and identifying the cause of battery failure. To this end, the development of technology to collect gas generated inside the secondary battery is important.

The following processes may be performed as methods for analyzing gas generated inside the secondary battery.

In order to collect an analysis target gas generated inside the secondary battery, a perforation hole is formed in a case of the secondary battery. The secondary battery with the perforation hole formed is accommodated in a gas diffusion space, which is a sealed space. After waiting for a predetermined period of time, the analysis target gas is diffused in the gas diffusion space. The analysis target gas diffused in the gas diffusion space is sampled in a separate sampling vessel or delivered to a gas analysis apparatus (e.g., GC-MS, etc.) to perform gas analysis.

In the above process, a method of accommodating the secondary battery entirely in the 'gas diffusion space' makes it difficult to provide separate analysis conditions (temperature, shock, vibration, etc.) to the secondary battery during the analysis process, and since the gas diffusion space requires a sufficiently larger volume than the secondary battery, the analysis target gas may be diluted more than necessary.

In order to improve this, there is a method of collecting the analysis target gas by closely contacting an apparatus for collecting gas with a surface of the case of the secondary battery with the perforation hole formed. However, even with this method, bends or the like may be formed in the case of the secondary battery during the perforating process, and it is difficult to expect perfect airtightness due to this phenomenon, and there are limitations in long-term analysis.

Therefore, a gas collecting or analysis apparatus that solves the above issues is required.

### Technical Goals

The present disclosure aims to provide a gas collecting apparatus for collecting gas generated inside a secondary battery in real time.

Further, the present disclosure is to provide a gas analysis apparatus for analyzing gas generated inside a secondary battery in real time while imposing various conditions to the secondary battery. Technical objects to be achieved by the present disclosure are not limited to the technical objects mentioned above, and other technical objects not mentioned may be clearly understood by those skilled in the art to which the present disclosure pertains from the description below.

### Technical Solutions

A gas collecting apparatus of the present disclosure is for collecting gas of a battery and includes:
a lower jig part having a battery penetration hole defined therein, wherein end parts of the lower jig part are open;
a sealing part having a closed loop shape located above the lower jig part; and
an upper jig part having a battery insertion groove defined therein and a gas diffusion space, wherein the upper jig part is coupled to the lower jig part and the sealing part is located in between the lower jig part and the upper jig part.

According to an aspect, a cylindrical battery may be slidably inserted into the battery penetration hole of the lower jig part, and the battery may be capable of idling within the lower jig part.

According to an aspect, the lower jig part may have a cylindrical shape extending in a vertical direction, the battery penetration hole may penetrate the lower jig part in the vertical direction, and a length of the lower jig part in the vertical direction may be shorter than a length of the battery to be analyzed in the vertical direction.

According to an aspect, the battery insertion groove may be defined in a surface of the upper jig part in contact with the sealing part and may have a cylindrical shape extending in the vertical direction, and wherein the battery insertion groove includes a first section having a first inner diameter and a second section having a second inner diameter, wherein the first inner diameter and the second inner diameter may be larger than an outer diameter of the battery, and the first inner diameter may be larger than the second inner diameter.

According to an aspect, the sealing part may have a ring shape having a predetermined thickness, the first inner diameter may be larger than a sum of the outer diameter of the battery and the thickness of the sealing part, the second inner diameter may be shorter than the sum of the outer diameter of the battery and the thickness of the sealing part, and the sealing part may be in close contact with a step located at a boundary of the first section and the second section.

According to an aspect, screw threads may be disposed on an inner circumferential surface of the first section of the battery insertion groove and an outer circumferential surface of the lower jig part, and the lower jig part may be screw-coupled to the battery insertion groove.

According to an aspect, the gas diffusion space may be located in an upper region of the second section of the battery insertion groove.

According to an aspect, the gas diffusion space may have a disk shape, and a diameter of the gas diffusion space may be smaller than a diameter of the analysis target battery.

According to an aspect, the upper jig part may further comprises: a gas delivery path connected to the gas diffusion space; and a carrier gas supply path connected to an auxiliary diffusion space, wherein the upper jig part includes the auxiliary diffusion space in a shape of an arc, wherein the auxiliary diffusion space has a predetermined width and is centered on a center of the gas diffusion space and is spaced apart from the gas diffusion space, and wherein the gas diffusion space and the auxiliary diffusion space may communicate by a plurality of carrier gas injection holes.

According to an aspect, a size of a central angle of the arc formed by the auxiliary diffusion space may range from 90° to 270°.

A gas analysis apparatus according to the present disclosure includes:
the gas collecting apparatus as described above,
a carrier gas supply unit configured to supply carrier gas to the gas diffusion space of the gas collecting apparatus through a carrier gas supply path;
a mass flow control unit configured to control a flow rate of the carrier gas; and
a gas analysis unit configured to analyze gas delivered through a gas delivery path from the gas diffusion space.

According to an aspect, the gas analysis apparatus may further include a condition imposing unit coupled to an exposed lower end part of the battery, wherein the battery may be inserted into the battery penetration hole of the lower jig part.

According to an aspect, the condition imposing unit may include a charging/discharging part configured to charge or discharge the battery.

According to an aspect, the condition imposing unit may further include one or more selected from a vibrating part, an impact part, a heater part, a cooling part, a pressurizing part, and a negative pressure part.

According to an aspect, the gas delivery path may include a manifold unit, and
the gas analysis unit may be connected to the manifold unit, and the gas analysis apparatus may further include a vacuum pump unit connected to the manifold unit, and a pressure measuring unit connected to the manifold unit.

### Advantageous Effects

A gas collecting apparatus of the present disclosure is for collecting gas generated inside a secondary battery in real time.

In the gas collecting apparatus of the present disclosure, a seal may be formed on a side surface other than a perforated surface of the secondary battery, thereby eliminating a leakage problem due to curvature of the perforated surface.

The gas collecting apparatus of the present disclosure may efficiently deliver an analysis target gas by allowing carrier gas to flow radially centering on a perforated hole of the secondary battery.

A gas analysis apparatus of the present disclosure may analyze gas generated inside a cylindrical secondary battery in real time while imposing various conditions to the secondary battery.

### Brief Description of the Drawings

FIG. 1 is a perspective view illustrating a gas collecting apparatus according to an aspect of the present disclosure.
FIG. 2 is a cross-sectional view taken along line A-A' of FIG. 1.
FIG. 3 illustrates an upper jig part according to an aspect of the present disclosure.
FIG. 4 is a cross-sectional view taken along line B-B' of FIG. 1.
FIG. 5 illustrates a gas analysis apparatus according to an aspect of the present disclosure.
FIG. 6 is a block diagram schematically illustrating a gas analysis apparatus according to another aspect of the present disclosure.

### Detailed Description

Hereinafter, aspects according to the present disclosure will be described in detail with reference to the accompanying drawings. In this process, the size or shape of the components shown in the drawings may be exaggerated for clarity and convenience of explanation. In addition, terms specifically defined in consideration of the configuration and operation of the present disclosure may vary according to the intentions or customs of users and operators. Definitions of these terms should be made based on the content throughout this specification.

In the description of the present disclosure, it should be noted that the orientation or positional relationship indicated by the terms such as "center", "upper", "lower", "left", "right", "vertical", "horizontal", "inner side", "outer side", "one surface", and "other surface" is based on the orientation or positional relationship shown in the drawing or the orientation or positional relationship normally arranged when using the product of the present disclosure, and it is intended only for explanation and brief description of the present disclosure, and is not to be construed as limiting the present disclosure as it does not suggest or imply that the device or element shown must necessarily be configured or operated in a specific orientation with a specific orientation.

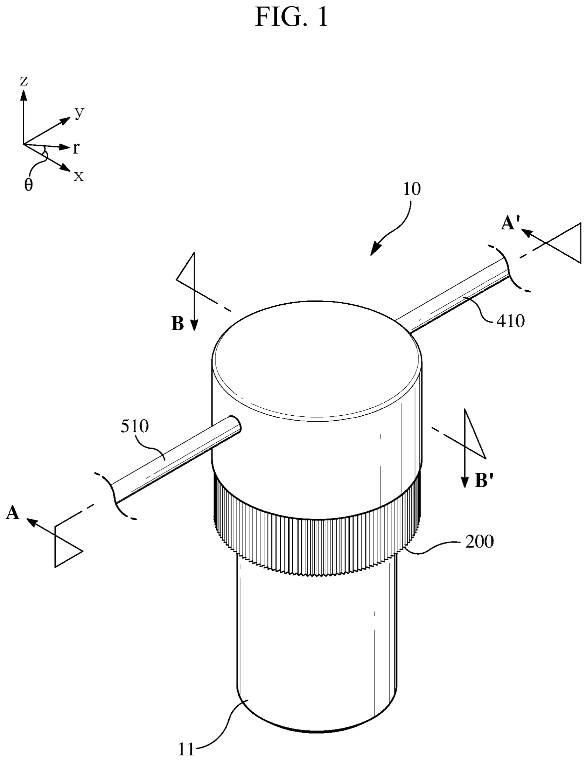

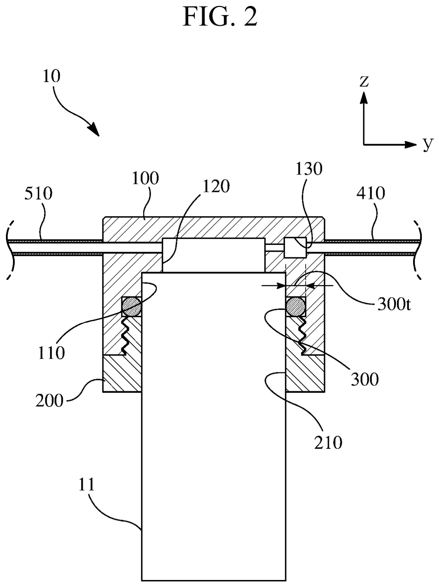

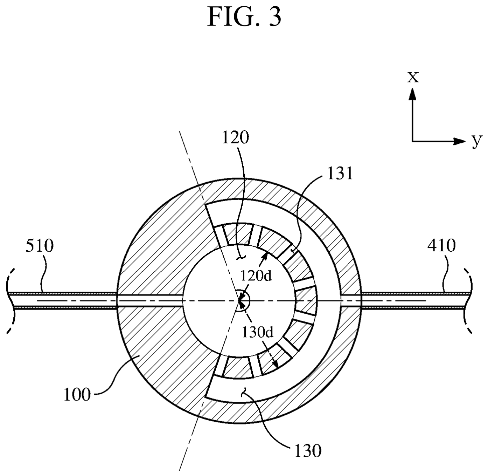

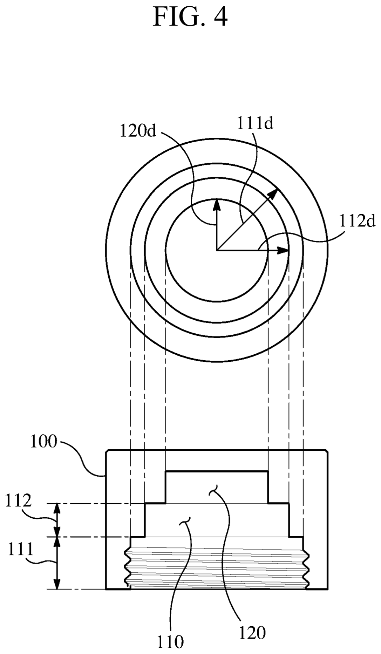

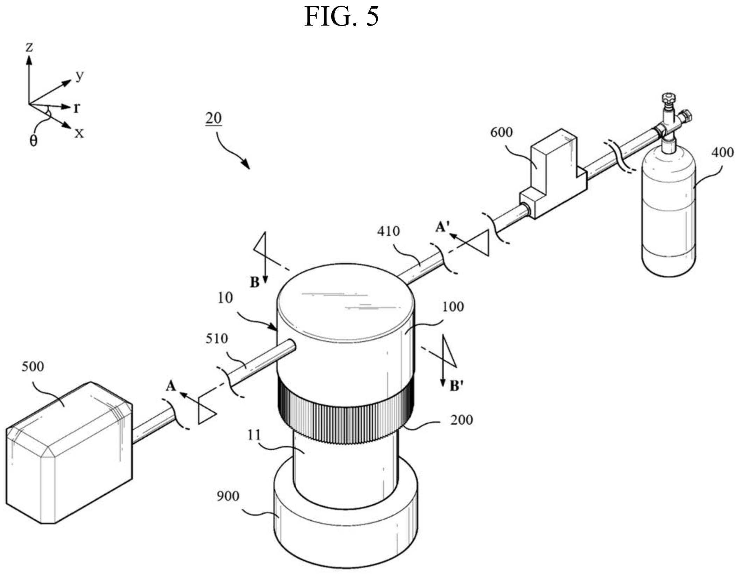

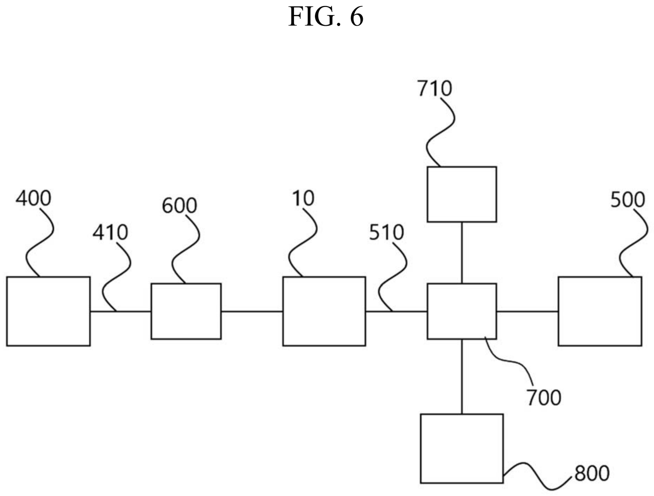

FIG. 1 is a perspective view illustrating a gas collecting apparatus 10 according to an aspect of the present disclosure. FIG. 2 is a cross-sectional view taken along line A-A' of FIG. 1. FIG. 3 is a conceptual diagram illustrating an upper jig part 100. FIG. 4 is a cross-sectional view taken along line B-B' of FIG. 1. FIG. 5 illustrates a gas analysis apparatus according to an aspect of the present disclosure. FIG. 6 is a block diagram illustrating a gas analysis apparatus according to another aspect of the present disclosure.

Hereinafter, with reference to FIGS. 1 to 6, the gas collecting apparatus 10 and a gas analysis apparatus 20 of the present disclosure will be described in detail.

The gas collecting apparatus 10 of the present disclosure may collect gas generated inside a secondary battery. Specifically, the secondary battery may be an analysis target battery 11, and the gas generated inside the secondary battery (an anode, a cathode, electrolyte, and the like, of the secondary battery) may be collected in real time while imposing physical conditions such as charging and discharging as well as temperature changes to the secondary battery.

In the gas collecting apparatus 10 of the present disclosure, the analysis target battery 11 may be a cylindrical secondary battery. The cylindrical secondary battery may be housed in a jelly roll shape in a state where an anode, a cathode, and a separator are laminated in a cylindrical case made of a rigid material. A cathode tab and an anode tab for electrically connecting the cathode and the anode to the outside may both be located at one end part of the secondary battery. In other words, one end part of the secondary battery may be an electrode side having a cathode tab and an anode tab, and the other end part of the secondary battery may be a non-electrode side without a cathode tab and an anode tab.

In order to collect gas, the non-electrode side may be perforated using a sharp tool to form a perforation hole. At this time, the surface of the non-electrode side may be distorted as the perforation hole is formed, thereby forming an irregular curve. Therefore, if a seal is formed directly on the corresponding non-electrode side surface, there is a concern that leakage may occur due to the irregular curve.

The gas collecting apparatus 10 of the present disclosure collects gas by covering the non-electrode side surface, but the seal is formed on the side surface of the battery, thereby stably forming the seal and maintaining it for a long time. In addition, since the gas collecting apparatus 10 of the present disclosure enables gas collecting while leaving a part of the analysis target battery 11 including the electrode side open to the outside, various conditions such as heating, cooling, shock, vibration, etc., as well as charging and discharging, may be imposed to the analysis target battery 11 during gas collecting.

In FIGS. 1 to 5, a z-axis direction may be a vertical direction. In the cylindrical coordinate system shown in FIG. 1, an r direction may be a first direction. In FIGS. 1 to 5, an x-axis direction may be a direction perpendicular to the vertical direction. In FIGS. 1 to 5, a y-axis direction may be a direction perpendicular to the vertical direction and the x-axis direction.

The analysis target battery 11 may be coupled with the gas collecting apparatus 10 in a state where the electrode side faces downward, the non-electrode side faces upward, and the longitudinal direction is parallel to the vertical direction.

As shown in FIGS. 1 and 2, the gas collecting apparatus 10 of the present disclosure includes:
a lower jig part 200 surrounding a side surface of the analysis target battery 11;
a sealing part 300 in a closed loop shape located above the lower jig part and into which the battery 11 is fitted; and
an upper jig part 100 covering the upper part of the battery 11, and coupled with the lower jig part 200 with the sealing part 300 in between;
a carrier gas supply path 410 configured to supply carrier gas to a gas diffusion space 120 provided inside the upper jig part 100; and
a gas delivery path 510 configured to deliver gas of the gas diffusion space 120 to a gas collecting vessel or a gas analysis apparatus.

The gas diffusion space 120 may be formed as a gas diffusion groove on a surface of the upper jig part 100 facing the battery 11.

A battery penetration hole 210 may be formed in the lower jig part 200, and the analysis target battery 11 may be inserted into the battery penetration hole 210, and the outer circumferential surface of the battery 11 may slide on the inner circumferential surface of the battery penetration hole 210, so that the battery 11 may be capable of idling within the lower jig part 200.

The lower jig part 200 is provided in a cylindrical shape extending in the vertical direction, and the battery penetration hole 210 may be formed to penetrate the lower jig part 200 in the vertical direction so that both end parts of the lower jig part 200 are open. The length of the lower jig part 200 in the vertical direction may be shorter than the length of the battery 11 in the vertical direction. More specifically, the length of the lower jig part 200 in the vertical direction may be shorter than the length of the battery 11 in vertical direction minus the depth of a battery insertion groove 110. Accordingly, the electrode side of the analysis target battery 11 may be completely exposed to the outside, and various conditions may be applied to the analysis target battery 11 while collecting gas.

A step may be formed along the circumferential direction on the outer circumferential surface of the lower jig part 200, and the outer diameter of the upper part of the lower jig part 200 may be formed smaller than the outer diameter of the lower part. The inner diameter of the lower jig part 200 may be formed to be almost similar to the outer diameter of the analysis target battery 11, but may have a length that causes almost no friction during idling.

A screw line may be formed on the upper part of the outer circumferential surface of the lower jig part 200, and a wrinkle extending in the vertical direction may be formed on the lower part. The user may rotate the lower jig part 200 while gripping it to screw-couple the lower jig part 200 to the upper jig part 100.

As illustrated in FIG. 3, a battery insertion groove 110 into which a part of the battery 11 is inserted may be formed on the bottom surface of the upper jig part 100. The battery insertion groove 110 may be in a cylindrical shape extending in the vertical direction, and may be formed with a first inner diameter 111d from the bottom surface entrance to a first section 111, and a second inner diameter 112d from the first section 111 to a second section 112, wherein the first inner diameter 111d and the second inner diameter 112d may be longer than the outer diameter of the battery 11, and the first inner diameter 111d may be longer than the second inner diameter 112d.

The sealing part 300 may be ring-shaped, and for example, the sealing part 300 may be an O-ring. The sealing part 300 has a predetermined thickness in the first direction (r direction in the cylindrical coordinate system). When this is referred to as a sealing thickness 300t, the length of the first inner diameter 111d may be longer than the sum of the outer diameter of the analysis target battery 11 and the sealing thickness 300t, and the length of the second inner diameter 112d may be shorter than the sum of the outer diameter of the battery 11 and the sealing thickness 300t.

The material of the sealing part 300 may be a chemically resistant elastic material, and may include one or more of silicone rubber, nitrile rubber, butadiene rubber, fluorine rubber, and ethylene propylene diene monomer rubber. The diameter (inner diameter or outer diameter) of the sealing part 300 may be smaller than the outer diameter of the battery 11 when it is not mounted on the battery 11, but when mounted on the analysis target battery 11, the elastic material may be stretched and completely adhered to the side surface of the battery 11. It is preferable that the material of the sealing part 300 be a material having a high coefficient of friction with the material of the case of the analysis target battery 11, because this may prevent the battery 11 from being separated from the gas collecting apparatus 10 during gas collecting. The sealing thickness 300t may be the length in the first direction when the sealing part 300 is mounted on the battery 11 and is stretched. The sealing part 300 may be in close contact with a ring-shaped step formed at the boundary between the first section 111 and the second section 112.

Screw threads may be formed on the inner circumferential surface of the first section 111 of the battery insertion groove 110 and the outer circumferential surface of the lower jig part 200, and the upper end part of the lower jig part 200 may be inserted into the battery insertion groove 110 and screw-coupled. As described above, by rotating the lower jig part 200 while the upper jig part 100 is fixed, the upper jig part 100 and the lower jig part 200 may be screw-coupled to each other.

As illustrated in FIG. 3, a gas diffusion space 120 may be formed in the upper region of the battery insertion groove 110, i.e., the upper region of the second section.

The shape of the gas diffusion space 120 may be formed in a disk shape, and the diameter 120d of the gas diffusion space 120 may be formed smaller than the diameter of the battery 11.

As illustrated in FIG. 4, inside the upper jig part 100, an auxiliary diffusion space 130 in the shape of an arc having an inner diameter 130d larger than the diameter 120d of the gas diffusion space 120 and centered on the center of the gas diffusion space 120 may be provided. In addition, the gas diffusion space 120 and the auxiliary diffusion space 130 may be communicated by a plurality of carrier gas injection holes 131.

The gas delivery path 510 may be connected to the gas diffusion space 120, and the carrier gas supply path 410 may be connected to the auxiliary diffusion space 130.

The size of the central angle of the arc formed by the auxiliary diffusion space 130 may range from 90° to 270°. A plurality of the carrier gas injection holes 131 may be formed and disposed to be spaced apart from each other at a uniform interval (angle). In this way, the carrier gas may be dispersedly injected radially, thereby achieving efficient gas delivery. At this time, if the size of the central angle is too large, discharge into the gas delivery path 510 may be hindered, and if the size of the central angle is too small, uniform gas diffusion in the gas diffusion space 120 may be hindered.

A gas analysis apparatus 20 using the gas collecting apparatus 10 as described above includes,
a carrier gas supply unit 400 configured to supply carrier gas to the gas diffusion space 120 of the gas collecting apparatus 10 through the carrier gas supply path 410;
a mass flow control unit 600 configured to control the flow rate of the carrier gas; and
a gas analysis unit 500 configured to analyze gas delivered from the gas diffusion space 120 through the gas delivery path 510.

FIG. 5 illustrates an example of a gas analysis apparatus 20 using the gas collecting apparatus 10 according to the present disclosure.

The gas analysis apparatus 20 of FIG. 5 may include a gas collecting apparatus 10 coupled to an upper end part of the analysis target battery 11; a condition imposing unit 900 coupled to a lower end part of the analysis target battery 11; a carrier gas supply unit 400 configured to supply carrier gas to a gas diffusion space formed inside the gas collecting apparatus 10; a carrier gas supply path 410 configured to deliver the carrier gas supplied by the carrier gas supply unit 400 to the gas diffusion space; a mass flow control unit 600 provided in the carrier gas supply path 410 and configured to control the supply amount of the carrier gas supplied to the gas diffusion space; a gas analysis unit 500 configured to receive and analyze analysis target gas from the gas diffusion space; and a gas delivery path 510 configured to deliver the analysis target gas of the gas diffusion space to the gas analysis unit 500.

The condition imposing unit 900 may be provided with a charging/discharging part configured to charge or discharge the analysis target battery 11.

The gas analysis apparatus 20 may be provided with the charging/discharging part capable of charging or discharging the battery, and may deliver gas generated under various charging (discharging) conditions in real time to the gas analysis unit 500 while changing the state of charge (SOC) of the analysis target battery 11.

The condition imposing unit 900 may further include one or more of a vibrating part, an impact part, a heater part, a cooling part, a pressurizing part, and a negative pressure part.

The vibration part may vibrate the analysis target battery 11 at a predetermined time cycle. For example, the vibration part may be a device that may transmit sound waves or ultrasonic waves to the battery 11.

The impact part may be capable of applying a physical impact to the analysis target battery 11. For example, the impact part may collide a rigid body with the battery 11 in an instantaneous period of time.

The heater part may supply a heat source to the analysis target battery 11. For example, the heater part may be a coil heater, an infrared heater, an induction heating heater, a dielectric heating heater, etc.

The cooling part may take heat energy from the analysis target battery 11. For example, the cooling part may be a chiller, liquid nitrogen, etc.

The pressurizing part may apply pressure to the surface of the analysis target battery.

The negative pressure part may be a vacuum chamber.

In addition, by being provided with the mass flow control unit 600, the gas analysis apparatus 20 may dilute the concentration of the analysis target gas injected into the gas analysis unit 500 to an appropriate level.

The gas analysis unit 500 may include one or more of a gas chromatography-mass spectroscopy (GC-MS), a gas chromatography-pulsed discharge detector (GC-PDD), a gas chromatography-thermal conductivity detector (GC-TCD), a gas chromatography-flame ionization detector (GC-FID), a Fourier transform infrared spectroscopy (FT-IR), and a Raman spectroscopy.

The gas delivery path 510 and the carrier gas supply path 410 may include a pipe, a hose, a tube, or the like for delivering gas.

The carrier gas supply unit 400 may be a gas bombe in which an inert gas is stored.

The mass flow control unit 600 may be a mass flow controller (MFC). The mass flow control unit 460 may control the injection amount of the carrier gas to make the gas concentration optimized for analysis by the gas analysis unit 500 and deliver the analysis target gas to the gas analysis unit 500.

FIG. 6 illustrates a configuration of a gas analysis apparatus according to another aspect of the present disclosure. The gas analysis apparatus illustrated in FIG. 6 may include the gas collecting apparatus 10 according to the present disclosure;
a carrier gas supply unit 400 connected to the carrier gas supply path 410;
a mass flow control unit 600 configured to control the supply amount of the carrier gas injected into the gas diffusion space through the carrier gas supply path 410;
a manifold unit 700 connected to the gas delivery path 510;
a vacuum pump unit 800 connected to the manifold unit 700;
a pressure measuring unit 710 connected to the manifold unit 700; and
a gas analysis unit 500 connected to the manifold unit 700.

The gas analysis unit 500 may be a gas chromatography-mass spectroscopy (GC-MS) device, a gas chromatography-pulsed discharge detector (GC-PDD) device, a gas chromatography-thermal conductivity detector (GC-TCD) device, a gas chromatography-flame ionization detector (GC-FID) device, or a Fourier transform infrared spectroscopy (FT-IR) device.

The gas delivery path 510 and the carrier gas supply path 410 may include a pipe, a hose, a tube, or the like for delivering gas.

The carrier gas supply unit 400 may be a gas bombe in which an inert gas is stored.

The manifold unit 700 may be a multi-pipe having a passage formed therein that functions as a pipe and a plurality of device connection ports on the outside.

The mass flow control unit 600 may be a mass flow controller (MFC).

The vacuum pump unit 800 may be selected from, for example, an oil rotary pump, a Roots pump, an oil diffusion pump, a turbo molecular pump, a cryopump, an ion pump, etc.

The pressure measuring unit 710 may be a pressure gauge.

Although aspects according to the present disclosure have been described above, they are only illustrative and those skilled in the art will understand that various modifications and aspects of equivalent range are possible therefrom. Therefore, the true technical protection scope of the present disclosure should be defined by the appended claims.

### <Explanation of Symbols>

10...Gas collecting apparatus
11...Analysis target battery
100...Upper jig part
110...Battery insertion groove
111...First section
111d...First inner diameter
112...Second section
112d...Second inner diameter
120...Gas diffusion space
120d...Diameter of gas diffusion space
130...Auxiliary diffusion space
130d...Inner diameter of auxiliary diffusion space
131...Carrier gas injection hole
200...Lower jig part
210...Battery penetration hole
300...Sealing part
300t...Sealing part thickness
400...Carrier gas supply unit
410...Carrier gas supply path
500...Gas analysis unit
510...Gas delivery path
600...Mass flow control unit
700...Manifold unit
710...Pressure measuring unit
800...Vacuum pump unit
900... Condition imposing unit

## Claims

1. A gas collecting apparatus configured to collect gas of a battery, comprising:
a lower jig part having a battery penetration hole defined therein, wherein end parts of the lower jig part are open;
a sealing part having a closed loop shape located above the lower jig part; and
an upper jig part having a battery insertion groove defined therein and a gas diffusion space, wherein the upper jig part is coupled to the lower jig part and the sealing part is located in between the lower jig part and the upper jig part.

2. The gas collecting apparatus of claim 1, wherein a cylindrical battery is slidably inserted into the battery penetration hole of the lower jig part, and the battery is capable of idling within the lower jig part.

3. The gas collecting apparatus of claim 2, wherein the lower jig part has a cylindrical shape extending in a vertical direction, the battery penetration hole penetrates the lower jig part in the vertical direction, and a length of the lower jig part in the vertical direction is shorter than a length of the battery to be analyzed in the vertical direction.

4. The gas collecting apparatus of claim 3, wherein the battery insertion groove is defined in a surface of the upper jig part in contact with the sealing part and has a cylindrical shape extending in the vertical direction, and wherein the battery insertion groove comprises a first section having a first inner diameter and a second section having a second inner diameter, and wherein the first inner diameter and the second inner diameter are larger than an outer diameter of the battery, and the first inner diameter is larger than the second inner diameter.

5. The gas collecting apparatus of claim 4, wherein the sealing part has a ring shape having a predetermined thickness, the first inner diameter is larger than a sum of the outer diameter of the battery and the thickness of the sealing part, the second inner diameter is shorter than the sum of the outer diameter of the battery and the thickness of the sealing part, and the sealing part is in close contact with a step located at a boundary of the first section and the second section.

6. The gas collecting apparatus of claim 4, wherein screw threads are disposed on an inner circumferential surface of the first section and an outer circumferential surface of the lower jig part, and the lower jig part is screw-coupled to the battery insertion groove.

7. The gas collecting apparatus of claim 4, wherein the gas diffusion space is located in an upper region of the second section of the battery insertion groove.

8. The gas collecting apparatus of claim 7, wherein the gas diffusion space has a disk shape, and a diameter of the gas diffusion space is smaller than diameter of the battery.

9. The gas collecting apparatus of claim 8, further comprising:
a gas delivery path connected to the gas diffusion space; and
a carrier gas supply path connected to an auxiliary diffusion space,
wherein the upper jig part includes the auxiliary diffusion space in a shape of an arc, wherein the auxiliary diffusion space has a predetermined width and is centered on a center of the gas diffusion space and is spaced apart from the gas diffusion space, and wherein the gas diffusion space and the auxiliary diffusion space communicate by a plurality of carrier gas injection holes.

10. The gas collecting apparatus of claim 9, wherein a size of a central angle of the arc formed by the auxiliary diffusion space ranges from 90° to 270°.

11. A gas analysis apparatus, comprising:
the gas collecting apparatus of claim 1;
a carrier gas supply unit configured to supply carrier gas to the gas diffusion space of the gas collecting apparatus through a carrier gas supply path;
a mass flow control unit configured to control a flow rate of the carrier gas; and
a gas analysis unit configured to analyze gas delivered through a gas delivery path from the gas diffusion space.

12. The gas analysis apparatus of claim 11, further comprising a condition imposing unit coupled to an exposed lower end part of the battery, wherein the battery is inserted into the battery penetration hole of the lower jig part.

13. The gas analysis apparatus of claim 12, wherein the condition imposing unit includes a charging/discharging part configured to charge or discharge the battery.

14. The gas analysis apparatus of claim 13, wherein the condition imposing unit further comprises one or more selected from a vibrating part, an impact part, a heater part, a cooling part, a pressurizing part, or a negative pressure part.

15. The gas analysis apparatus of claim 11, further comprising:
a manifold unit connected to the gas analysis unit;
a vacuum pump unit; and
a pressure measuring unit,
wherein the gas delivery path includes the manifold unit, the vacuum pump unit is connected to the manifold unit, and the pressure measuring unit connected to the manifold unit.
